# FASCICULE DE BREVET EUROPEEN

(11) **EP 0 766 542 B1**
(45) Date de publication et mention de la délivrance du brevet: **22.07.1998**
(21) Numéro de dépôt: 95923383.4
(22) Date de dépôt: 14.06.1995
(51) Int. Cl.: A61F 5/01

(54) **STRUCTURE DE MAINTIEN POUR L'ARTICULATION DE LA CHEVILLE**
STRUKTUR ZUM HALTEN DES SPRUNGGELENKES
STRUCTURE FOR HOLDING THE ANKLE JOINT

(30) Priorité: 14.06.1994 FR 9407503
(43) Date de publication de la demande: 09.04.1997
(73) Titulaire: Rhenter, Jean-Luc, 1275 Cheserex (CH)
(72) Inventeur: Rhenter, Jean-Luc, 1275 Cheserex (CH)
(74) Mandataire: Vuillermoz, Bruno
(86) Numéro de dépôt international: FR9500780
(87) Numéro de publication internationale: WO9534257

(56) Documents cités:
- WO-A-87/07498
- WO-A-93/02644
- US-A- 1 381 290
- US-A- 5 069 202

## Description

L'invention concerne un structure de maintien, plus particulièrement destinée à l'articulation de la cheville. Une telle structure de maintien au sens de l'invention peut s'utiliser de manière isolée, c'est à dire dont la mise en place s'effectue indépendamment de toute chaussure, mais peut également s'intégrer au sein d'une chaussure, plus spécifiquement d'une chaussure de sport.

La complexité des mouvements dont l'articulation de la cheville est le siège, rend celle-ci particulièrement sujette à de nombreux traumatismes, de sorte que depuis longtemps déjà, on cherche, notamment à titre préventif, à maintenir et soutenir cette articulation. Ceci est tout particulièrement opportun dès lors qu'une activité, notamment sportive, sollicite sévèrement cette articulation.

Deux tendances se sont développées à ce jour, en vue de favoriser à titre préventif le soutien et le maintien de cette articulation. La première consiste à réaliser une chaussure montante comportant des renforts rigides. De telles chaussures, si elles laissent relativement libres l'articulation tibio-tarsienne, en revanche, affectent quelque peu la liberté de mouvement inhérente à l'articulation sous astragalienne et qui plus est, affectent pour le moins le confort de l'utilisateur.

On a également proposé des chaussures moins rigides, donc plus confortables, mais dont la contention opérée au niveau de la cheville, tant au niveau de l'articulation tibio-tarsienne que de l'articulation sous astragalienne, est insuffisante pour réaliser un réel maintien de la cheville en général, ne permettant pas d'éviter les traumatismes ou de limiter ceux-ci lorsqu'ils apparaissent.

On connait également d'autres solutions curatives pour le traitement des entorses de cheville, et de manière plus générale des laxités de la cheville. Outre la méthode ancienne consistant à procéder à une intervention chirurgicale en vue de remplacer ou suturer le ou les ligaments défectueux, qui s'avère longue et douloureuse, on a également proposé l'utilisation de bandes élastiques de contention, mieux connues sous l'expression en langue anglaise "strapping", qui consiste à entourer en la serrant l'articulation de la cheville dans une telle bande. Cependant, une telle bande, outre qu'elle assure une contention notoirement insuffisante de la cheville, s'avère de mise en oeuvre longue et fastidieuse avant et après son utilisation. Enfin, on a quelquefois recours à l'utilisation d'une chevillière, constituée d'une structure en tissu souple et élastique, enfilée à l'instar d'une chaussette autour de la cheville, mais qui présente l'inconvénient de n'assure qu'un médiocre maintien de la cheville.

Le document WO-A-93/02644 décrit un dispositif de maintien pour l'articulation de la cheville qui comprend toutes les caractéristiques du préambule de la revendication 1.

L'objet de l'invention est de proposer une structure de maintien pour l'articulation de la cheville, susceptible d'être utilisée tant à titre préventif qu'à titre curatif, permettant de s'affranchir de ces différents inconvénients, ladite structure étant susceptible d'être utilisée à titre d'orthèse, voire être intégrée directement au sein d'une chaussure, notamment de sport, montante ou non.

Cette structure selon la revendication 1 comporte trois parties articulées entre elles, respectivement :
- une première partie rigide, dite tige, destinée à venir entourer partiellement et prendre appui contre l'extrémité inférieure de la jambe de l'utilisateur et, se subdivisant dans sa zone terminale en deux branches venant se terminer au niveau des deux malléoles dudit utilisateur ;
- une seconde partie dite coque pédieuse, destinée à entourer le pied au niveau de la voute plantaire ;
- et enfin une troisième partie, dite coque de liaison ou coque sous-astragalienne, sensiblement en forme de V, destinée à s'articuler respectivement au niveau des malléoles à la tige, aux deux extrémités des branches du V, de telle sorte à permettre le débattement de ladite tige dans le plan sagittal de l'articulation tibio-tarsienne, et d'autre part, au niveau de l'extrémité supérieure de la coque pédieuse à la pointe du V, de telle sorte à permettre les mouvements d'éversion et d'inversion de la coque pédieuse par rapport à l'ensemble constitué par la tige et la coque de liaison.

En d'autres termes, l'invention consiste à réaliser une structure de trois éléments, articulés les uns par rapport aux autres, permettant de par leur conception, d'assurer les différents mouvements selon les degrés de liberté naturels de l'articulation de la cheville, tant par rapport à l'articulation sous-astragalienne que par rapport à l'articulation tibio-tarsienne, tout en maintenant lesdites articulations de telle sorte à limiter, sinon annuler tout risque de traumatisme articulaire à ce niveau, ce que le dispositif et autres chaussures connues à ce jour ne permettent pas d'obtenir.

En effet, tous ces dispositifs, s'ils assurent certes une certaine contension de l'articulation de la cheville, n'autorisent qu'un mouvement de ladite articulation dans le plan sagittal de l'articulation tibio-tarsienne, et affectant de manière significative voire rédhibitoire, le mouvement de l'articulation sous-astragalienne, c'est à dire les mouvements d'inversion et d'éversion du pied.

Selon une forme avantageuse de l'invention, l'articulation de la coque de liaison sur la coque pédieuse est positionnée au niveau d'un bossage ménagé au sein de l'extrémité supérieure de ladite coque pédieuse, bossage sur lequel vient coopérer une zone de forme complémentaire de la coque de liaison, articulée au niveau d'un axe d'articulation traversant les deux coques, permettant la rotation de ladite coque de liaison par rapport à la coque pédieuse.

Qui plus est, en fonction de l'étendue de la lumière traversante, ménagée au sein de la coque de liaison au niveau de la zone d'articulation, on peut augmenter l'amplitude du débattement relatif desdites coques l'une par rapport à l'autre.

Selon une caractéristique avantageuse de l'invention, on intègre deux inserts, respectivement au sein de la coque pédieuse et de la coque de liaison au niveau de leur zone de coopération, lesdits inserts étant destinés à coopérer entre eux afin de permettre les mouvements d'éversion et d'inversion de la coque pédieuse par rapport à l'ensemble tige - coque de liaison, ainsi que le mouvement de débattement de la tige dans le plan sagittal de l'articulation tibio-tarsienne, l'insert intégré au sein de ladite coque de liaison comportant une lumière traversante, coopérant avec un certain jeu avec une saillie issue de l'insert intégré dans la coque pédieuse pour justement permettre le mouvement relatif des éléments constituant la structure de maintien selon ces différents degrés de liberté, ce jeu étant cependant limité au moyen d'un écrou de blocage, venant se visser sur l'extrémité filetée de ladite saillie.

La manière dont l'invention peut être réalisée et les avantages qui en découlent, de même que les caractéristiques supplémentaires de l'invention ressortiront mieux de l'exemple de réalisation qui suit donné à titre indicatif et non limitatif à l'appui des figures annexées.
La figure 1 est une représentation schématique en perspective et vue de côté d'une structure de soutien pour pied droit selon l'invention.
La figure 2 est une vue en perspective de face de la structure de soutien de la figure 1.
La figure 3 est une vue de détail du système de fixation et d'articulation de la coque de liaison sur la coque pédieuse.
La figure 4 est une représentation schématique en coupe de ladite articulation.
La figure 5 est une représentation schématique latérale externe de l'articulation de la cheville.
La figure 6 est une représentation schématique en perspective éclatée de l'organe de liaison de la coque pédieuse avec la coque de liaison, dont les figures 7 et 8 sont respectivement une vue en section et une vue de dessus, lorsque ledit organe est assemblé.

Bien que plus particulièrement décrite en liaison avec une orthèse de cheville, et ainsi que déjà dit, l'invention ne saurait se limiter à cette seule application et est tout à fait intégrable, notamment au sein d'une chaussure de sport.

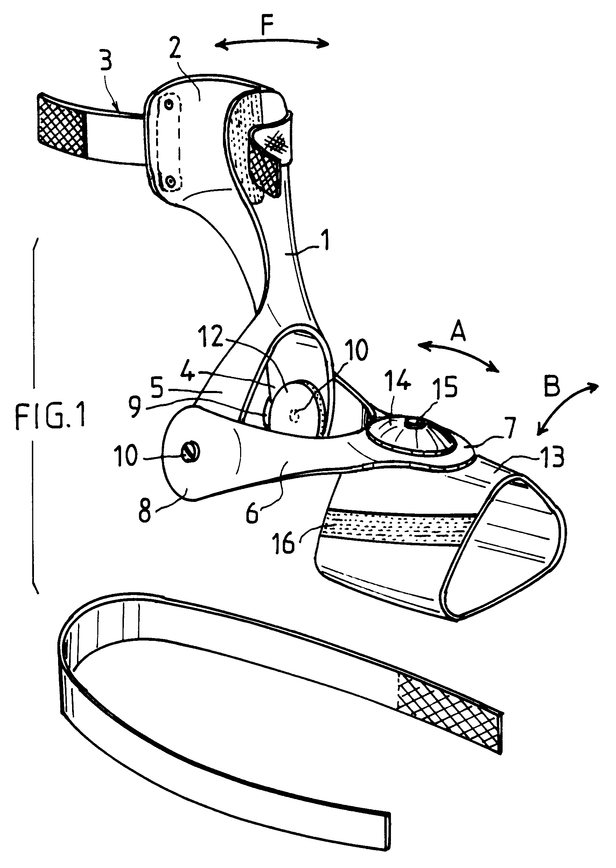

On a représenté au sein de la figure 1 une vue schématique latérale en perspective de cette orthèse pour un pied droit. Cette orthèse ou structure de soutien comporte trois éléments fondamentaux, respectivement :
- une tige rigide (1) ;
- une coque pédieuse (13) ;
- une coque de liaison (6), dite coque sous-astragalienne, articulée respectivement à l'extrémité inférieure de la tige (1) et sur le dessus de la coque pédieuse (13).

Ces différents éléments sont réalisés en un matériau rigide, tel que par exemple en fibres de carbone, fibres de verre, fibre de polyaramide, voire en un mélange fibres de carbone - fibres de polyaramide (KEVLAR : marque déposée), voire même en matière plastique thermoformable. Ce dernier matériau présente l'avantage de permettre la réalisation d'une coque standard avec une possibilité de modification de la morphologie locale de l'un de ces éléments pour une adaptation partielle au pied de l'utilisateur.

Dans l'optique d'une recherche accrue du confort, la totalité de la coque sous-astragalienne (6), est réalisée en mélange carbone - KEVLAR, afin de lui conférer une certaine souplesse en regard de la rigidité des fibres de carbone utilisées isolément, afin notamment de permettre une adaptation anatomique de la dite coque (6) pour le passage du tendon jambier antérieur.

Ainsi qu'on peut l'observer sur la figure 1, la tige (1) enveloppe partiellement l'extrémité inférieure de la jambe de l'utilisateur, et se termine en zone inférieure selon deux branches, dont les extrémités (4, 5) viennent sensiblement coïncider avec les malléoles.

Cette tige (1) est réversiblement fixée à la jambe de l'utilisateur au moyen d'une courroie (3) élastique, se fermant par coopération d'un système boucles-crochets, selon le système bien connu sous la marque déposée VELCRO.

Cette tige (1) est articulée avec la coque sous-astragalienne (6) au niveau des malléoles (10), selon un axe d'articulation (11), légèrement incliné, correspondant sensiblement à l'axe anatomique inter-malléoles. De fait, l'axe légèrement incliné des malléoles constitue le premier axe d'articulation de l'orthèse entre la tige (1) et la coque sous-astragalienne (6). De la sorte, on reproduit exactement le mouvement dynamique de l'articulation tibio-tarsienne, selon la flèche F de la figure 1.

Parallèlement et en vue d'optimiser le confort de l'utilisateur, la face interne des extrémités (4, 5) de la tige (1) reçoit au niveau des malléoles un coussin (12), d'épaisseur réduite, par exemple réalisé en polystyrène ou en néoprène, destiné à àdoucir le contact des malléoles de l'utilisateur avec la tige.

La coque sous astragalienne (6) a sensiblement la forme d'un V, dont l'extrémité des deux branches, respectivement (8) et (9) vient s'articuler au niveau des malléoles à l'extérieur de la tige (1). La pointe du V (7) vient quant à elle s'articuler au niveau de la coque pédieuse (13), et plus spécifiquement à son extrémité supérieure.

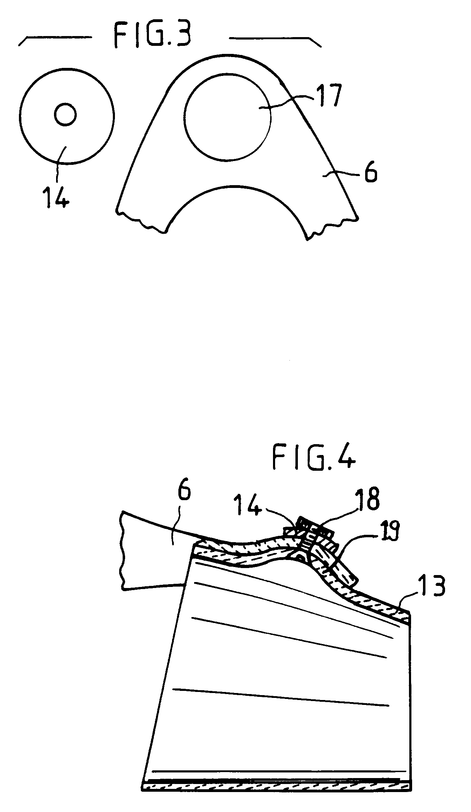

Selon une première forme de réalisation de l'invention, la coque pédieuse (13) présente au niveau de la zone d'articulation de la coque sous astragalienne (6), un bossage (19) (voir figure 4) sur lequel vient coopérer l'extrémité (7) de ladite coque sous-astragalienne (6), présentant à ce niveau une forme correspondante, et ce, dans le but de favoriser la rotation de l'extrémité de la coque sous astragalienne au niveau de la coque pédieuse.

Plus spécifiquement, l'axe d'articulation (18), autour duquel est susceptible de tourner la coque sous-astragalienne (6), vient pincer d'une part une rondelle externe (14), de forme sensiblement circulaire, puis l'extrémité de la coque sous-astragalienne (6) et enfin le bossage (19) de la coque pédieuse (13).

Afin de permettre un plus grand débattement de la coque sous-astragalienne (6) lors de sa rotation autour et sur le bossage (19) de la coque pédieuse (13) (selon flèches A et B de la figure 1), l'orifice qu'elle comporte, afin de permettre le passage de l'axe d'articulation (18) est transformé en une lumière traversante (17), mais dont le diamètre demeure cependant inférieur au diamètre de la rondelle (14). On conçoit de la sorte que l'on puisse augmenter à loisir le débattement possible en rotation de la coque sous-astragalienne et de la tige qui lui est directement articulée, par rapport à la coque pédieuse. On peut ainsi avantageusement, et de manière simple faire varier le degré de débattement d'éversion et d'inversion de l'ensemble tige-coque sous astragalienne par rapport à la coque pédieuse, et partant de l'articulation de la cheville en jouant sur les dimensions de cette lumière (17).

Dans une forme de réalisation particulière de l'invention, on peut avantageusement encliqueter au niveau de la lumière (17), un élément intermédiaire de forme correspondant à la forme du bossage (19) de la coque pédieuse (13), afin de réduire le diamètre de ladite lumière (17), et ce, pour limiter l'amplitude des degrés de liberté de rotation de ladite coque sous-astragalienne par rapport à la coque pédieuse.

Selon une autre caractéristique de l'invention, l'axe d'articulation (18) de la coque sous-astragalienne (6) par rapport à la coque pédieuse (13) est orienté sensiblement selon le cône sous astragalien (26) défini ci-après, et plus spécifiquement selon la génératrice (27) dudit cône sous-astragalien. On admet cependant une tolérance de plus ou moins 20 ° de l'orientation de cet axe dans le plan sagittal de l'articulation tibio-tarsienne par rapport à la référence constituée par la génératrice (27) de ce cône sous-astragalien.

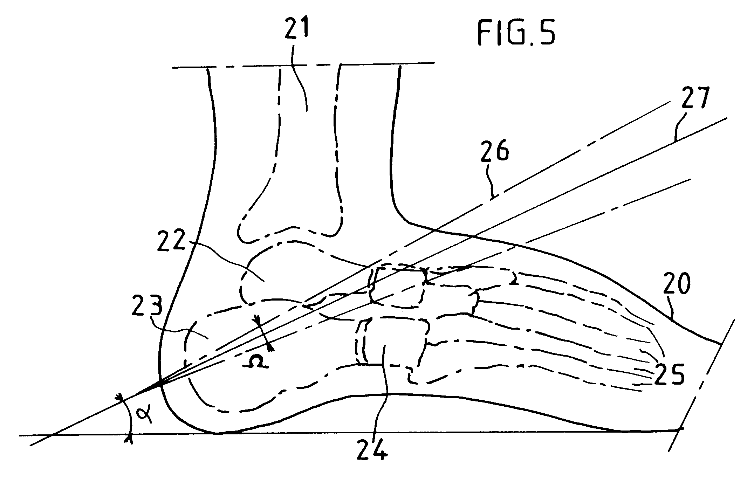

Il va être défini ci-après ce que l'on entend par cône sous-astragalien, et ce, à l'aide de la figure 5, qui représente une vue latérale schématique des os de la cheville.

De manière connue, l'extrémité inférieure du tibia (21) repose sur l'astragale (22), reposant elle-même sur le calcanéum (23) ou os du talon. En amont par rapport à l'astragale et au calcanéum, on retrouve différents os, notamment le cuboïde (24) et les métatarsiens (25).

L'astragale et le calcanéum sont intimement reliés au moyen de ligaments permettant les mouvements de l'articulation sous astragalienne, selon un mouvement d'inversion et un mouvement d'éversion de la cheville. De manière connue, l'inversion de la cheville correspond à un mouvement associant une combinaison de mouvements dans les trois plans de l'espace :
- respectivement dans le plan horizontal selon une rotation interne dirigée vers la face interne du pied opposé, et dont l'amplitude maximum est de l'ordre de 30 ° par rapport à une position d'aplomb correspondant à l'alignement de l'axe longitudinal du pied avec une parallèle au plan de symétrie de l'être humain,
- dans le plan frontal, un mouvement de roulis interne d'une amplitude maximale de 25 ° environ,
- et dans le plan sagittal d'une flexion dite plantaire d'une amplitude de 10 ° environ.

De la même manière, on définit par l'éversion de la cheville, un ensemble de trois mouvements associant dans les trois plans de l'espace une rotation externe d'amplitude maximum de 30 ° dans le plan horizontal, un roulis externe dans le plan frontal et une flexion dite dorsale dans le plan sagittal d'amplitude environ 10 °.

Les différentes études anatomiques effectuées, notamment par le Demandeur, ont pu mettre en évidence que les déplacements et mouvements de l'articulation sous-astragalienne, notamment, et des mouvements d'inversion et d'éversion sont limités et circonscrits à une enveloppe géométrique assimilable à un cône de révolution (26), dénommé cône sous-astragalien, dont le sommet S est situé sensiblement au niveau du tiers inférieur de la hauteur du calcanéum en arrière de celui-ci tel que représenté sur la figure 5. La génératrice (27) du cône (26) présente une inclinaison α par rapport au plan horizontal sur lequel repose le pied, dont la valeur est comprise entre 20 et 50 °.

Enfin, le cône (26) est défini par son angle d'ouverture ou angle au sommet Ω, dont la valeur moyenne varie entre 15 et 30 ° en fonction de l'âge des individus et de leurs particularités anatomiques.

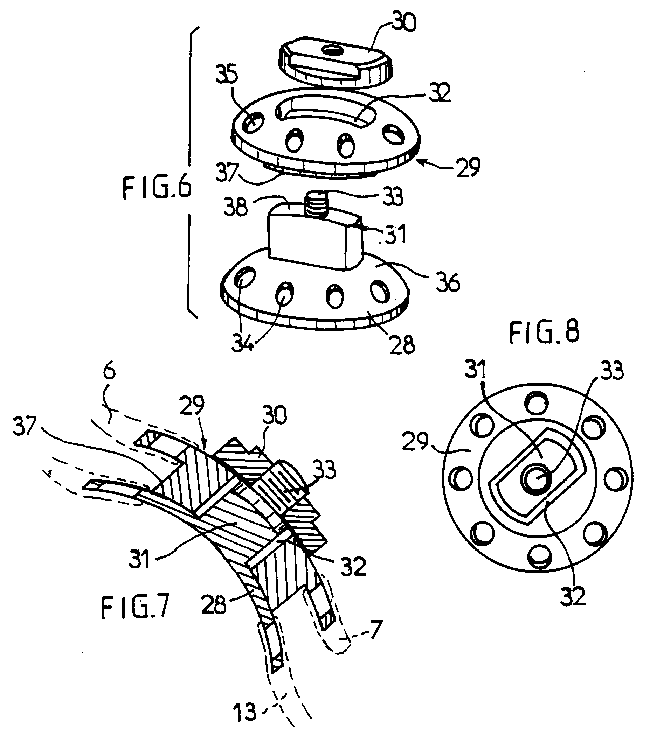

Selon une autre forme de réalisation de l'invention plus particulièrement décrite en liaison avec les figures 6 à 8, l'articulation de la coque sous-astragalienne (6) avec la coque pédieuse (13) s'effectue toujours au niveau de l'extrémité (7) de ladite coque (6), mais par l'intermédiaire d'inserts, par exemple métalliques (28, 29), respectivement intégrés au sein de la coque pédieuse (13) et de la coque sous-astragalienne (6, 7). De fait, et afin de faciliter cette intégration, lesdits inserts (28, 29), de forme circulaire, sont munis sur leur périphérie d'orifices traversants (34, 35), destinés à optimiser l'ancrage des matériaux constitutifs desdites coques lors de leur moulage.

L'insert (28), intégré au sein de la coque pédieuse (13), est bombé et présente une symétrie de révolution, la convexité étant dirigée vers l'extérieur, c'est à dire en direction du dessus de l'articulation. Avantageusement, l'axe de révolution de l'insert est aligné avec la génératrice (27) du cône sous-astragalien (26) aux tolérances près déjà mentionnées.

Cet insert (28) comporte sur sa face convexe (36), une saillie radiale (31), orientée selon l'axe de révolution de l'insert et s'étendant symétriquement de part et d'autre dudit axe. Cette saillie (31) est de section transversale sensiblement rectangulaire, dont les deux petits côtés sont arrondis. Enfin, elle se prolonge, selon l'axe de révolution de l'insert par une tige filetée (33) à partir de son extrémité supérieure (38), avantageusement de forme bombée.

L'insert (29), intégré au sein de l'extrémité (7) de la coque sous-astragalienne (6), est également de forme bombée, dont la convexité est également dirigée vers le dessus de l'articulation. Tout comme l'insert (28), il présente une symétrie de révolution, dont l'axe de révolution est alignée avec la génératrice (27) du cône (26) aux tolérances près.

Cet insert (29) présente une base (37), de diamètre inférieure mais de convexité de même degré que la convexité de la face convexe (36) de l'insert (28), sur laquelle elle est destinée à prendre appui. En outre, la base (37) et l'insert (29) sont percés d'une lumière traversante (32), dont la forme correspond à la forme de la saillie (31) de l'insert (28), mais dont les dimensions sont supérieures, et liées par exemple avec celles de la saillie (31) selon des rapports homothétiques, ainsi qu'on peut l'obsever sur la figure 8.

La saillie (31) est destinée à coopérer avec la lumière (32) dans le cadre de l'articulation de la coque sous-astragalienne (6) avec la coque pédieuse (13). Afin de maintenir les deux inserts (28, 29) en coopération étroite, un écrou (30) vient se visser sur la tige filetée (33) prolongeant la saillie (31). Cet écrou (30), présente avantageusement une face inférieure concave, propre à prendre appui sur la face supérieure convexe (38) de l'extrémité supérieure de la saillie (31), dont la longueur est légèrement supérieure à la hauteur de la lumière (32), pour ainsi permettre de bloquer ledit écrou selon une position propre à permettre le libre débattement selon les différents degrés de liberté déjà mentionnés de l'insert (29) par rapport à l'insert (28), et partant de la coque sous- astragalienne (6) par rapport à la coque pédieuse (13). Bien entendu, l'écrou (30) est de dimensions supérieures à celles de la lumière (32). Compte tenu de la forme particulière de la lumière (32) et de la saillie (31), l'orthèse peut permettre les débattements selon les différents degrés de liberté requis.

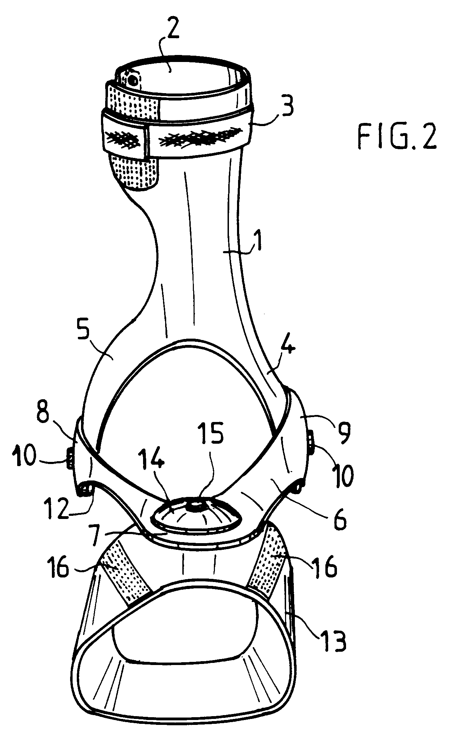

Selon une autre caractéristique de l'invention, l'orthèse est associée à un chausson (non représenté), réalisé par exemple en néoprène, préalablement chaussé par l'utilisateur avant mise en place de l'orthèse. Ce chausson en néoprène comporte une courroie passant au niveau du talon, et destiné à venir se fixer au moyen d'un système boucles-crochets du type bien connu sous la marque déposée VELCRO sur la coque pédieuse (13), et dont on a représenté sur les figures 1 et 2 les bandes complémentaires (16), destinées à coopérer avec ladite courroie, et ce, afin d'aboutir à la coaptation du calcanéum dans la coque pédieuse. Ce chausson peut être remplacé par un revêtement mousse collé.

De la sorte, la mise en place de l'orthèse suppose au préalable le chaussage du chausson en néoprène. Puis on procède à la fixation de l'orthèse, en prenant tout d'abord bien soin d'enfiler à fond le pied au sein de la coque pédieuse (13), au niveau de laquelle on ajuste le plus fermement possible la courroie solidaire du talon du chausson en néoprène. Enfin, on fixe la tige (1) sur la jambe, en serrant la courroie qui lui est associée.

On conçoit ainsi la rapidité de mise en place d'une telle orthèse, simplifiant drastiquement les procédures connues jusqu'alors, qui par ailleurs n'aboutissent pas à une contention efficace de la cheville, et n'assurent pas la possibilité d'un débattement selon tous les degrés de liberté anatomiques de cette articulation.

Parallèlement, cette procédure de mise en place est simplifiée, si cette orthèse est directement intégrée au sein d'une chaussure, notamment montante. En effet, dans ce cas, il n'y a plus de chausson en néoprène, la coaptation du pied au sein de la coque pédieuse étant directement assurée par le talon de la chaussure.

Cette orthèse présente de nombreux avantages, et notamment, celui de maintenir sous contension les deux articulations respectivement tibio-tarsienne et sous-astragalienne, tout en autorisant, voire en les limitant légèrement, les différents mouvements desdites articulations, selon leur degre de liberté anatomique naturelle. Parallèlement, de par sa conception, une telle structure peut être mise en place même avec des chaussures de sport, voire de ski de fond.

## Revendications

1. Structure de maintien pour l'articulation de la cheville, susceptible d'être utilisée tant à titre préventif qu'à titre curatif, comportant trois parties articulées entre elles, respectivement :
- une première partie rigide (1), dénommée tige, destinée à venir entourer partiellement et prendre appui contre l'extrémité inférieure de la jambe de l'utilisateur et, se subdivisant dans sa zone terminale en deux branches (4, 5) venant se terminer au niveau des deux malléoles de la cheville dudit utilisateur ;
- une seconde partie dite coque pédieuse (13) ;
- et enfin une troisième partie, caractérisée en ce que la troisième partie, dite coque sous-astragalienne (6), sensiblement en forme de V, destinée à s'articuler respectivement au niveau des malléoles à la tige (1), aux deux extrémités (8, 9) des branches du V, de telle sorte à permettre le débattement de ladite tige (1) dans le plan sagittal de l'articulation tibio-tarsienne, et d'autre part, au niveau de l'extrémité supérieure de la coque pédieuse (13), qui est destinée à entourer le pied au niveau de la voute plantaire à la pointe du V, de telle sorte à permettre les mouvements d'éversion et d'inversion de la coque pédieuse (13) par rapport à l'ensemble constitué par la tige (1) et la coque sous-astragalienne (6).

2. Structure de maintien pour l'articulation de la cheville selon la revendication 1, ***caractérisée*** en ce que l'articulation de la coque sous-astraglienne (6) sur la coque pédieuse (13) est positionnée au niveau d'un bossage (19) ménagé au sein de l'extrémité supérieure de ladite coque pédieuse, bossage sur lequel vient coopérer une zone de forme complémentaire de la coque sous-astraglienne (6), articulée au niveau d'un axe d'articulation (18) traversant les deux coques, permettant la rotation de ladite coque (6) par rapport à la coque pédieuse (13).

3. Structure de maintien pour l'articulation de la cheville selon la revendication 2, ***caractérisée*** en ce que la zone de la coque sous-astragalienne (6) coopérant avec l'axe d'articulation (18) et le bossage (19) de la coque pédieuse (13), est percée d'une lumière traversante (17), l'articulation comportant en outre une rondelle (14), également raversée par l'axe d'articulation (18), venant prendre appui sur ladite coque sous-astragalienne (6), afin d'assurer le maintien dudit axe (18).

4. Structure de maintien pour l'articulation de la cheville selon la revendication 3, ***caractérisée*** en ce que la lumière traversante (17) ménagée au sein de la coque sous-astragalienne (6), est susceptible de recevoir un élément intermédiaire de forme correspondant à la forme du bossage (19) de la coque pédieuse (13), fixé réversiblement sur la périphérie de ladite lumière, notamment par encliquetage, de telle sorte à réduire le diamètre de ladite lumière (17), et ce, pour limiter l'amplitude des degrés de liberté de rotation de ladite coque sous-astragalienne par rapport à la coque pédieuse.

5. Structure de maintien pour l'articulation de la cheville selon l'une des revendications 2 à 4, ***caractérisée*** en ce que l'axe d'articulation (18) est orienté selon la génératrice (27) du cône sous-astragalien (26), défini comme étant l'enveloppe géométrique intégrant l'ensemble des mouvements d'éversion et d'inversion de la cheville, avec une tolérance de plus ou moins 20 ° de l'orientation de l'axe (18) dans le plan sagittal de l'articulation tibio-tarsienne par rapport à la référence constituée par la génératrice (27) dudit cône sous-astragalien (26).

6. Structure de maintien pour l'articulation de la cheville selon la revendication 1, ***caractérisée*** en ce que l'articulation de la coque sous-astraglienne (6) sur la coque pédieuse (13) s'effectue au moyen d'inserts (28, 29), intégrés au sein desdites coques (6, 13) :
- un premier insert (28), intégré au sein de la coque pédieuse (13), de forme bombée et présentant une symétrie de révolution, la convexité étant dirigée vers le dessus de ladite articulation, comportant sur sa face convexe (36), une saillie radiale (31), orientée selon l'axe de révolution de l'insert et s'étendant symétriquement de part et d'autre dudit axe ;
- un second insert (29), intégré au sein de l'extrémité (7) de la coque sous-astragalienne (6), de forme bombée et présentant une symétrie de révolution, la convexité étant dirigée vers le dessus de l'articulation, et présentant une base (37), de convexité de même degré que la convexité de la face convexe (36) de l'insert (28), sur laquelle elle est destinée à prendre appui, la base (37) et l'insert (29) étant percés d'une lumière traversante (32), dont la forme correspond à la forme de la saillie (31) de l'insert (28), mais de dimensions supérieures, la saillie (31) de l'insert (28) étant destinée à coopérer avec ladite lumière (32).

7. Structure de maintien pour l'articulation de la cheville selon la revendication 6, ***caractérisée*** en ce que les deux inserts (28, 29) sont maintenus en coopération étroite au moyen d'un écrou (30), de dimensions supérieures à celles de la lumière (32), ledit écrou venant se visser sur une tige filetée (33) prolongeant la saillie (31).

8. Structure de maintien pour l'articulation de la cheville selon la revendication 7, ***caractérisée*** en ce que l'écrou (30) présente une face inférieure concave, propre à prendre appui sur la face supérieure convexe (38) de l'extrémité supérieure de la saillie (31), dont la longueur est légèrement supérieure à la hauteur de la lumière (32), pour ainsi permettre de bloquer ledit écrou selon une position propre à permettre le libre débattement de l'insert (29) par rapport à l'insert (28), et partant de la coque sous-astragalienne (6) par rapport à la coque pédieuse (13) selon les mouvements d'éversion et d'inversion, ainsi que dans le plan sagittal de l'articulation tibio-tarsienne.

9. Structure de maintien pour l'articulation de la cheville selon l'une des revendications 6 à 8, ***caractérisée*** en ce que les axes de révolution des inserts (28, 29) sont alignés avec la génératrice (27) du cône sous-astragalien (26) défini comme étant l'enveloppe géométrique intégrant l'ensemble des mouvements d'éversion et d'inversion de la cheville, avec une tolérance de plus ou moins 20° de l'orientation des dits axes dans le plan sagittal de l'articulation tibio-tarsienne par rapport à la référence constituée par la génératrice (27) dudit cône sous-astragalien (26).

10. Structure de maintien pour l'articulation de la cheville selon l'une des revendications 6 à 9, ***caractérisée*** en ce que la saillie (31) et la lumière traversante (32) sont de forme sensiblement rectangulaire, dont les deux petits côtés sont arrondis, et en ce que les dimensions de la saillie (31) et de la lumière traversante (32) sont liées entre elles selon des rapports homothétiques.

11. Structure de maintien pour l'articulation de la cheville selon l'une des revendications 1 à 10, ***caractérisée*** en ce qu'elle comprend en outre un chausson, notamment réalisé en néoprène, destiné à être préalablement chaussé par l'utilisateur avant la mise en place de ladite structure, ledit chausson étant pourvu d'une courroie solidarisé en zone postérieure, et destinée à venir se fixer de manière réversible sur le dessus de la coque pédieuse (13), afin d'induire la coaptation du pied de l'utilisateur au sein de ladite coque pédieuse.

12. Structure de maintien pour l'articulation de la cheville selon l'une des revendications 1 à 10, ***caractérisée*** en ce qu'elle est intégrée au sein d'une chaussure, la coaptation du pied de l'utilisateur au sein de la coque pédieuse (13) étant alors directement induite par le talon de la chaussure.

13. Structure de maintien pour l'articulation de la cheville selon l'une des revendications 1 à 12, ***caractérisée*** en ce qu'elle est réalisée en un matériau choisi dans le groupe comprenant les fibres de carbone, les fibres de verre, les fibres de polyaramide, ou un mélange de celles-ci, et les matières plastiques thermoformables.

## Claims

1. Support structure for the ankle joint, which can be used preventively and curatively, comprising three parts which are articulated to one another, respectively :
- a rigid first part (1), referred to as the upper, intended to partially surround and bear against the lower end of the user's leg and subdivided in its end region into two branches (4, 5) which end at the two malleoli of said user's ankle ;
- a second part (13), referred to as the pedal shell (13) ;
- and, finally, a third part,
characterized in that said third part (6), referred to as the sub-astragalar shell, which is substantially V-shaped and is intended to be articulated respectively, at the malleoli to the upper (1), at the two ends (8, 9) of the branches of the V, so as to allow displacement of said upper (1) in the sagittal plane of the tibiotarsal articulation and, on the other hand, to the upper end of the pedal shell (13), which is intended to surround the foot at the plantar arch at the tip of the V, so as to allow eversion and inversion movements of the pedal shell (13) relative to the assembly consisting of the upper (1) and the sub-astragalar shell (6).

2. Support structure for the ankle joint according to Claim 1, characterized in that the articulation of the sub-astragalar shell (6) to the pedal shell (13) is positioned at a boss (19) formed within the upper end of said pedal shell, on which boss a complementarily shaped region of the sub-astragalar shell (6) interacts, which region is articulated by an articulation pin (18) passing through the two shells, allowing said shell (6) to rotate relative to said pedal shell (13).

3. Support structure for the ankle joint according to Claim 2, characterized in that the region of the sub-astragalar shell (6) which co-operates with the articulation pin (18) and the boss (19) of the pedal shell (13) is pierced by a through-hole (17), the articulation furthermore including a washer (14), through which the articulation pin (18) also passes and which bears on said sub-astragalar shell (6) in order to hold said pin (18).

4. Support structure for the ankle joint according to Claim 3, characterized in that the through-hole (17) formed in the sub-astragalar shell (6) can accommodate an intermediate element which has a shape corresponding to the shape of the boss (19) of the pedal shell (13) and is fixed reversibly on the periphery of said hole, in particular by snap-fastening, so as to reduce the diameter of said hole (17) in order to limit the amplitude of the degrees of freedom of said sub-astragalar shell to rotate relative to the pedal shell.

5. Support structure for the ankle joint according to one of Claims 2 to 4, characterized in that the articulation pin (18) is oriented along the generatrix (27) of the sub-astragalar cone (26), the latter being defined as the geometrical envelope incorporating all the eversion and inversion movements of the ankle, with a tolerance of plus or minus 20° in the orientation of the pin (18) in the sagittal plane of the tibiotarsal articulation relative to the reference constituted by the generatrix (27) of said sub-astragalar cone (26).

6. Support structure for the ankle joint according to Claim 1, characterized in that the sub-astragalar shell (6) is articulated to the pedal shell (13) by means of inserts (28, 29) incorporated in said shells (6, 13) :
- a first insert (28),which is incorporated in the pedal shell (13), is of domed shape, and has symmetry of revolution, the convexity being directed toward the top of said articulation, and has a radial projection (31) on its convex face (36), this projection being oriented along the axis of revolution of the insert and extending symmetrically on either side of said axis ;
- a second insert (29) which is incorporated in the end (7) of the sub-astragalar shell (6), is of domed shape, and has symmetry of revolution, the convexity being directed toward the top of the articulation, and has a base (37), with the same degree of convexity as the convex face (36) of the insert (28) on which it is intended to bear, the base (37) and the insert (29) being pierced by a through-hole (32) whose shape corresponds to the shape of the projection (31) of the insert (28) but has larger dimensions, the projection (31) of the insert (28) being intended to interact with said hole (32).

7. Support structure for the ankle joint according to Claim 6, characterized in that the two inserts (28, 29) are kept in close interaction by means of a nut (30) whose dimensions are greater than those of the hole (32), said nut being screwed onto a threaded rod (33) extending the projection (31).

8. Support structure for the ankle joint according to Claim 7, characterized in that the nut (30) has a concave lower face which can bear on the convex upper face (38) of the upper end of the projection (31), the length of the latter being slightly greater than the height of the hole (32) in order thus to make it possible to immobilize said nut in a position which can allow free displacement of the insert (29) relative to the insert (28), and thereby of the sub-astragalar shell (6) relative to the pedal shell (13) in eversion and inversion movements as well as in the sagittal plane of the tibiotarsal articulation.

9. Support structure for the ankle joint according to one of Claims 6 to 8, characterized in that the axes of revolution of the inserts (28, 29) are aligned with the generatrix (27) of the sub-astragalar cone (26), the latter being defined as the geometrical envelope incorporating all the eversion and inversion movements of the ankle, with a tolerance of plus or minus 20° in the orientation of said axes in the sagittal plane of the tibiotarsal articulation relative to the reference constituted by the generatrix (27) of said sub-astragalar cone (26).

10. Support structure for the ankle joint according to one of Claims 6 to 9, characterized in that the projection (31) and the through-hole (32) are of substantially rectangular shape, in which the two small sides are rounded, and in that the dimensions of the projection (31) and of the through-hole (32) are related to one another by homothetic ratios.

11. Support structure for the ankle joint according to one of Claims 1 to 10, characterized in that it furthermore comprises a sock, in particular made of neoprene, intended for the user to put on before said structure is fitted, said sock being provided with a strap which is secured in the posterior region and is intended to be fixed reversibly on the top of the pedal shell (13) in order to induce coaptation of the user's foot within said pedal shell.

12. Support structure for the ankle joint according to one of Claims 1 to 10, characterized in that it is incorporated in a shoe or boot, coaptation of the user's boot within the pedal shell (13) then being induced directly by the heel of the shoe or boot.

13. Support structure for the ankle joint according to one of Claims 1 to 12, characterized in that it is made of a material chosen from the group comprising carbon fibers, glass fibers, polyaramid fibers or a mixture thereof, and thermoformable plastics.

## Patentansprüche

1. Struktur zum Halten des Sprunggelenks, welche zum vorsorgenden und heilenden Einsatz verwendbar ist, mit drei aneinander angelenkten Teilen, nämlich:
- einem ersten steifen Teil (1), Schaft genannt, welches dazu vorgesehen ist, das untere Ende des Beines des Benutzers teilweise zu umgreifen und sich dagegen abzustützen und welches sich in seiner Endzone in zwei Schenkel (4, 5) unterteilt, die im Bereich der beiden Knöchel des Fußes des genannten Benutzers enden;
- einem zweiten Teil, Fußschale (13) genannt,
- und schließlich einem dritten Teil, **dadurch gekennzeichnet**, daß der dritte Teil, Unterknöchelschale (6) genannt, der im wesentlichen eine V-Form aufweist, dazu vorgesehen ist, einerseits im Bereich der Knöchel an dem Schaft (1) an den beiden Enden (8, 9) der Schenkel des Vs angelenkt zu sein, so daß er das Verschwenken des genannten Schafts (1) in der Sagittalebene des oberen Sprunggelenks ermöglicht ist, und andererseits im oberen Endbereich der Fußschale (13), welche dazu vorgesehen ist, den Fuß im Bereich der Fußwölbung zu umfassen, an der Spitze des Vs angelenkt ist, so daß Eversions- und Inversionsbewegungen der Fußschale (13) in bezug auf die durch den Schaft (1) und die Unterknöchelschale (6) gebildete Einheit ermöglicht sind.

2. Struktur zum Halten des Sprunggelenks nach Anspruch 1, **dadurch gekennzeichnet**, daß die Anlenkung der Unterknöchelschale (6) an der Fußschale (13) im Bereich einer Wölbung (19) positioniert ist, die in dem oberen Endbereich der genannten Fußschale eingeformt ist, wobei die Wölbung mit einer komplementär geformten Zone der Unterknöchelschale (6) zusammenwirkt, welche an einer Gelenkachse (18), welche die beiden Schalen durchragt, angelenkt ist, so daß das Drehen der genannten Schale (6) in bezug auf die Fußschale (13) ermöglicht ist.

3. Struktur zum Halten des Sprunggelenks nach Anspruch 2, **dadurch gekennzeichnet**, daß die mit der Gelenkachse (18) und der Wölbung (19) der Fußschale (13) zusammenwirkende Zone der Unterknöchelschale (6) von einer durchgehenden Öffnung (17) durchbrochen ist, wobei die Anlenkung weiterhin eine ebenfalls von der Gelenkachse (18) durchragte Unterlegscheibe (14) aufweist, welche sich auf die Unterknöchelschale (6) abstützt, um den Halt der genannten Achse (18) zu gewährleisten.

4. Struktur zum Halten des Sprunggelenks nach Anspruch 3, **dadurch gekennzeichnet**, daß die in das Material der Unterknöchelschale (6) eingearbeitet durchgehende Öffnung (17) zur Aufnahme eines Zwischenelementes geeignet ist, welches eine der Wölbung (19) der Fußschale (13) entsprechende Form aufweist und lösbar an der Peripherie der genannten Öffnung insbesondere durch Einrasten befestigt ist, so daß der Durchmesser der genannten Öffnung (17) reduziert wird, um die Amplitude des Rotationsfreiheitgrades der genannten Unterknöchelschale in Bezug auf die Fußschale zu begrenzen.

5. Struktur zum Halten des Sprunggelenks nach einem der Ansprüche 2 bis 4, **dadurch gekennzeichnet**, daß die Gelenkachse (18) nach der Erzeugenden (27) des Unterknöchel-Kegels (26) ausgerichtet ist, welcher als geometrische Einhüllende diffiniert ist, die die Gesamtheit der Eversions- und Inversionsbewegungen des Knöchels umfaßt, mit einer Toleranz von plus oder minus 20° von der Orientierung der Achse (18) in der Sagittalebene des oberen Sprunggelenkes in Bezug auf die durch die Erzeugende (27) des genannten Unterknöchel-Kegels (26) gebildeten Referenz.

6. Struktur zum Halten des Sprunggelenks nach Anspruch 1, **dadurch gekennzeichnet**, daß die Anlenkung der Unterknöchelschale (6) an der Fußschale (13) mittels Einsatzteilen (28, 29) bewirkt ist, welche innerhalb der genannten Schalen (6, 13) integriert sind, nämlich:
- einem ersten, innerhalb der Fußschale (13) integrierten Einsatzteil (28) von gewölbter Form, welches eine Rotationssymmetrie aufweist, wobei die Wölbung zum oberen Abschnitt der genannten Anlenkung hin gerichtet ist und wobei seine konvexe Oberfläche (36) einen radialen Vorsprung (31) aufweist, der entlang der Rotationsachse des Einsatzteils ausgerichtet ist und sich symmetrisch zur einen und zur anderen Seite der genannten Achse erstreckt;
- einem zweiten, innerhalb des Endes (7) der Unterknöchelschale (6) integrierten Einsatzteil (29) von gewölbter Form, welches eine Rotationssymmetrie aufweist, wobei die Wölbung zum oberen Abschnitt der Anlenkung hin gerichtet ist, und welches eine Grundfläche (37) aufweist, deren Wölbung das gleich Maß wie die Wölbung der gewölbten Fläche (36) des Einsatzteiles (28), welches zur Abstützung gegen diese Grundfläche vorgesehen ist, aufweist, wobei die Grundfläche (37) und das Einsatzteil (29) von einer durchgehenden Öffnung (32) durchbrochen sind, deren Form der Form des Vorsprungs (31) des Einsatzteils (28) entspricht, allerdings mit größeren Abmessungen, wobei der Vorsprung (31) des Einsatzteils (28) dazu vorgesehen ist, mit der genannten Öffnung (32) zusammenzuwirken.

7. Struktur zum Halten des Sprunggelenks nach Anspruch 6, **dadurch gekennzeichnet**, daß die beiden Einsatzteile (28, 29) im engen Zusammenschluß zueinander mittels einer Schraubmutter (30) gehalten sind, deren Abmessung größer sind, als die der Öffnung (32), wobei die genannte Schraubmutter auf einen Gewindezapfen (33), der den Vorsprung (31) verlängert, aufgeschraubt ist.

8. Struktur zum Halten des Sprunggelenks nach Anspruch 7, **dadurch gekennzeichnet**, daß die Schraubmutter (30) eine untere konkave Fläche aufweist, welche zur Anlage gegen die obere konvexe Fläche (38) des oberen Endes des Vorsprungs (31) vorgesehen ist, dessen Länge etwas größer ist, als die Höhe der Öffnung (32), um so zu ermöglichen, daß die genannte Schraubmutter in einer Position blockiert wird, die geeignet ist, das freie Verschwenken des Einsatzteil (29) bezüglich des Einsatzteils (28) und somit das freie Verschwenken der Unterknöchelschale (6) bezüglich der Fußschale (13) gemäß den Eversions- und Inversionsbewegungen sowie in der Sagittalebene des oberen Sprunggelenkes zu ermöglichen.

9. Struktur zum Halten des Sprunggelenks nach einem der Ansprüche 6 bis 8, **dadurch gekennzeichnet**, daß die Rotationsachsen der Einsatzteile (28, 29) auf die Erzeugende (27) des Unterknöchel-Kegels (26) ausgerichtet ist, welcher als geometrische Einhüllende definiert ist, die die Gesamtheit der Eversions- und Inversionsbewegungen des Knöchels umfaßt mit einer Toleranz von plus oder minus 20° der Orientierung der genannten Achsen in der Sagittalebene des oberen Sprunggelenkes in Bezug auf die durch die Erzeugende (27) des genannten Unterknöchel-Kegels (26) gebildeten Referenz.

10. Struktur zum Halten des Sprunggelenks nach einem der Ansprüche 6 bis 9, **dadurch gekennzeichnet**, daß der Vorsprung (31) und die durchgehenden Öffnung (32) eine im wesentlichen rechteckige Form aufweisen, deren beide kurze Seiten gerundet sind, und daß die Dimensionen des Vorsprunges (31) und der durchgehenden Öffnung (32) untereinander gemäß homothetischen Beziehungen gekoppelt sind.

11. Struktur zum Halten des Sprunggelenks nach einem der Ansprüche 1 bis 10, **dadurch gekennzeichnet**, daß sie weiterhin eine Socke insbesondere aus Neopren umfaßt, welche dazu vorgesehen ist, durch den Benutzer vor dem Anordnen der genannten Struktur angezogen zu werden, wobei die genannte Socke mit einem im hinteren Bereich befestigten Gurt versehen ist, der dazu vorgesehen ist, lösbar an der Fußschale (13) befestigt zu werden, um den Halt des Fußes des Benutzers innerhalb der genannten Fußschale zu gewährleisten.

12. Struktur zum Halten des Sprunggelenks nach einem der Ansprüche 1 bis 10, **dadurch gekennzeichnet**, daß sie innerhalb eines Schuhs integriert ist, wobei der Halt des Fußes des Benutzers innerhalb der Fußschale (13) direkt durch den Fersenbereich des Schuhs gewährleistet ist.

13. Struktur zum Halten des Sprunggelenks nach einem der Ansprüche 1 bis 12, **dadurch gekennzeichnet**, daß sie aus einem Material hergestellt ist, welches aus der Gruppe ausgewählt ist, die Kohlefasern, Glasfasern, Polyaramid-Fasern oder eine Mischung hiervon, sowie warmformbare Kunststoffe umfaßt.
